# EUROPEAN PATENT SPECIFICATION

(11) **EP 3 313 295 B1**
(45) Date of publication and mention of the grant of the patent: **18.10.2023**
(21) Application number: 16815084.5
(22) Date of filing: 16.06.2016
(51) Int. Cl.: A61B 13/00, A61B 1/24

(54) **TONGUE ANTERIORIZER AND METHOD OF OPERATING SAME**
ZUNGENHERVORHOLER UND VERFAHREN ZUR BEDIENUNG DAVON
DISPOSITIF POUR ANTÉRIORISER LA LANGUE ET SON PROCÉDÉ DE FONCTIONNEMENT

(30) Priority: 26.06.2015 TW 104120860; 19.08.2015 TW 104127061; 09.05.2016 US 201615149468
(43) Date of publication of application: 02.05.2018
(73) Proprietor: Hsu, Tzu-Li, Princeton, NJ 08540 (US)
(72) Inventor: Hsu, Tzu-Li, Princeton, NJ 08540 (US)
(74) Representative: Becker Kurig & Partner Patentanwälte mbB
(86) International application number: PCT/US2016/037789
(87) International publication number: WO 2016/209702

(56) References cited:
- EP-A2- 1 133 330
- EP-A2- 2 568 871
- EP-B1- 1 133 330
- EP-B1- 2 568 871
- WO-A1-2013/036891
- CN-U- 201 710 390
- CN-U- 201 782 778
- US-A- 4 610 243
- US-A- 5 467 763
- US-A- 5 667 481
- US-A- 5 743 853
- US-A- 6 003 510
- US-A- 6 045 499
- US-A1- 2001 034 474
- US-A1- 2001 039 949
- US-A1- 2007 289 591
- US-A1- 2012 271 118
- US-B2- 6 901 928

## Description

### BACKGROUND OF THE INVENTION

### 1. Field of the Invention

The invention relates to a tongue anteriorizer capable of moving the tongue anteriorly and then lifting it to open the laryngopharyngeal space for upper airway management.

### Description of Related Art

Endotracheal intubation is the process of inserting the endotracheal tube into the trachea. It is the most important procedure for general anesthesia and respiratory resuscitation. Because of variance in oral anatomy, it can be very difficult to do and become "difficult intubation." When that happens, it may cause damage to the airway. If it failed completely, the patient will be in a disaster situation.

There are four groups of medical device for endotracheal intubation, namely, laryngoscope, video laryngoscope, video stylet, and fiberoptic scope.

Laryngoscope is the most common device used for endotracheal intubation. After inserting it into the mouth, it pushes the tongue and mandible forward at about 45-degree angle to open the temporomandibular joint (TMJ) and the laryngopharyngeal space. However, this method is not easy to use on small chin, big tongue, and obese patient because the open space in the oral cavity is small, and it is not easy for laryngoscope to have enough space to work efficiently. For experienced physicians, the success rate for endotracheal intubation is about 90 to 95%. Further, endotracheal intubation failure may injure tissues of the mouth, or even causes the life of a patient.

Video laryngoscope improves on the laryngoscope by adding the video camera. It provides better visibility but does not solve the problems of the laryngoscope.

Video stylet is inside the endotracheal tube and is guided forward by its video image. Because it does not have functionality to open the space, the secretion of the mouth can make it very difficult to see. Pushing it forward, it may move the tongue together and change the normal anatomy around the larynx. When this happens, it is very easy to be disoriented and fails the intubation.

The diameter of fiberoptic scope is smaller and it has a suction function. Its success rate is the best among all intubation devices. However, the learning period is long, and it is time consuming, i.e., very often it will take more than one try to finish the procedure. It is also expensive and easily damaged.

US2007/289591 A1, which document represents the most relevant prior art and forms the basis for the preamble of claim 1, discloses an endotracheal tube exchanger (ETX) includes a tube slide portion having an elongated lengthwise dimension having a first end and a second end, a relatively narrow width dimension having a curved cross section, and a generally arcuate shape having inner and outer peripheries wherein the curved cross section forms a concavity along the inner periphery, and a handle portion extending outwardly from the outer periphery of the first end of said tube slide portion. Associated methods describe the use of the endotracheal tube exchanger which does not require insertion of the exchanger device within the endotracheal tube to be changed or within the new replacement endotracheal tube.

US6003510 discloses a hand tool for facilitating the introduction of a laryngeal mask into a patient. A hand tool insertable into a patient, the hand tool for engaging a laryngeal mask and having a pivot region. The hand tool engages the laryngeal mask, and when the hand tool is pivoted, the hand tool raises the mask from the back of the throat to permit proper insertion of the mask into the patient.

CN201782778 discloses a tongue depressor, which belongs to the field of medical instruments. The multifunctional tongue depressor is characterized in that a strip-shaped sheet is bent and then connected with a tongue depressor via a transitional inclined plate. The multifunctional tongue depressor is simple in structure, easy to be manufactured and convenient in use, can be used for cleaning surfaces of tongues when in disease examination, eliminates incentive of oral cavity diseases and is favorable for physical health.

EP2568871 A2 discloses a laryngoscope insertion section having a curved superior surface and a curved inferior surface, and a channel extending from the proximal end the channel having an inferior internal surface with a greater curvature than the curvature of the inferior surface. The insertion section is compatible with laryngoscope hardware optimised for indirect viewing, yet enables direct viewing. Additionally, the distance between the inferior and superior surfaces is at a maximum within the intermediate portion, and enables the dimensions of the proximal and distal portions to be minimized. Thus, the intermediate portion, which is located in the patient's oral cavity in use, is provided with greatest depth and therefore strength where the greatest forces are received, whereas the distal and proximal portions are of reduced dimensions to as to minimize trauma to the patient's airway and mouth areas, respectively, in use. Further structural features providing the insertion section with improved strength, with a minimum of material and size, are also disclosed.

EP1133330 A2 discloses a laryngeal airway device for sealing against the laryngeal opening includes an air tube with proximal and distal ends and a sealing member attached to the distal end. The sealing member includes a coupler for coupling the device to an introducer. Complementing the laryngeal airway device is an introducer that includes a track for receiving the coupler of the laryngeal airway device and guiding the sealing member to a sealing position with respect to the laryngeal inlet. The introducer may include an epiglottic engager on a distal end to engage the epiglottis and retain it while the sealing member is being tracked to engagement with the laryngeal inlet.

WO2013036891 discloses the present invention relates to a tongue depressor, In depressor for particular, the present invention relates to a tongue depressor designed to be utilized when the clinician is positioned behind the head of the patient, for example, during anesthesia in surgery where insertion or removal of an airway device into the patient is necessary. The depressor comprises an anatomical bend, gripping surface for holding the depressor, a tissue grabbing surface, and a barrel end at the distal end of the grabbing and moving tissue, such as the tongue.

CN201710390 discloses a novel tongue depressor, wherein the front end of a tongue depression section is in downward and smooth transition bending; a circular arc central angle of a circular arc section is 45-90 degrees, and the circular arc radius is 2-3cmm; the cross section of the circular arc section has waveform structure; the tongue depressor is made of high-temperature-resistant materials; as the front end of the novel tongue depressor adopts the structure in line with the physiology shape of the root of the human tongue, the tongue of a patient can be better attached to the tongue depressor, and two sides of the tongue of the patient can be prevented from upward curling up in diagnosis, thus being beneficial to the observation on interior of an oral cavity by a doctor; and simultaneously, the front end surface of the tongue depressor is downward bent, thus being capable of preventing the contraction of the tongue of the patient in diagnosis and improving the diagnosis efficiency. As being made of high-temperature-resistant materials, the novel tongue depressor can be sterilized at high temperature, and can be repeatedly used, thus saving resources.

All the problems from the above intubation devices come from not being able to open the oral airway properly. Thus, the need for improvement still exists.

### SUMMARY OF THE INVENTION

Thus, the present invention is a tongue anteriorizer. The invention is defined in claim 1.

### BRIEF DESCRIPTION OF THE DRAWINGS

FIG. 1 is a perspective view of a tongue anteriorizer
FIG. 2 is a side elevation of the tongue anteriorizer;
FIG. 3A is a side elevation view of the tongue anteriorizer made in a predetermined shape of 180-degree;
FIG. 3B is a side elevation view of a tongue anteriorizer made in a predetermined shape of 210-degree;
FIG. 3C is a side elevation view of a tongue anteriorizer made in a predetermined shape of 150-degree;
FIG. 3D is a side elevation view of a tongue anteriorizer made in a predetermined shape of 135-degree;
FIG. 3E is a side elevation view of a tongue anteriorizer made in a predetermined shape of 120-degree;
FIGS. 4A, 4B, 4C, 4D, and 4E are side views showing steps of inserting the tongue anteriorizer of FIG. 3A, into the base of the posterior part of the tongue of a patient, respectively;
FIG. 5 is a side view showing of inserting the tongue anteriorizer of FIG. 3B into the oral part of a patient;
FIG. 6 is a perspective view of a tongue anteriorizer
FIG. 7 is a perspective view of the second configuration of a tongue anteriorizer of FIG. 6;
FIG. 8 is an exploded view of a tongue anteriorizer according to an embodiment of the invention in he exploded perspective view;
FIG. 9 is a perspective view of a tongue anteriorizer of FIG. 8 in the combination state;
FIG. 10 is a perspective view of a first configuration of a tongue
FIG. 11 is a perspective view of the second configuration of the tongue anteriorizer of FIG. 10;
FIG. 12 is a perspective view of a tongue anteriorizer
FIG. 13 is a perspective view of a tongue anteriorizer
FIG. 14 is a perspective view of a tongue anteriorizer and
FIG. 15 is a perspective view of a tongue anteriorizer.

### DETAILED DESCRIPTION

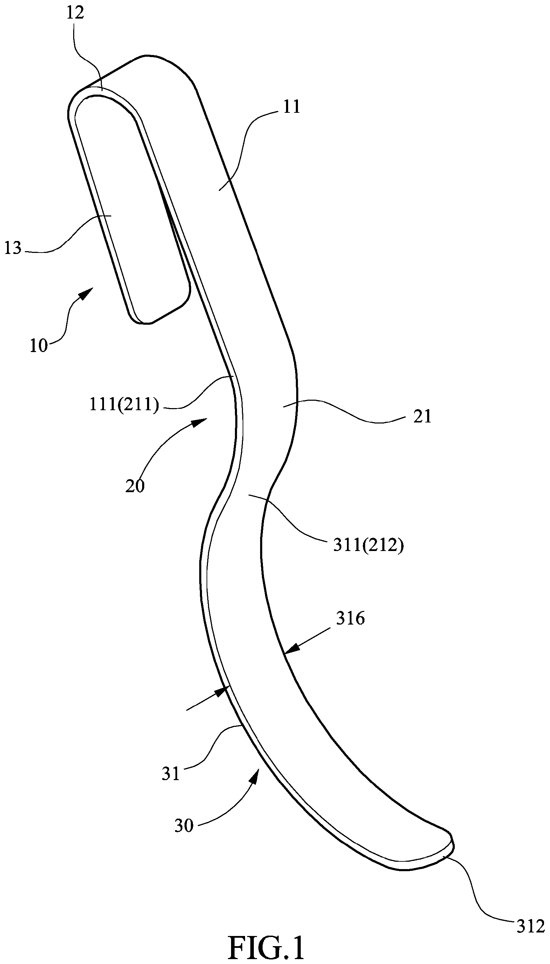

Referring to FIG. 1, a tongue anteriorizer comprises a handle 10, a connecting part 20, and a tongue lifting part 30 as discussed in detail below.

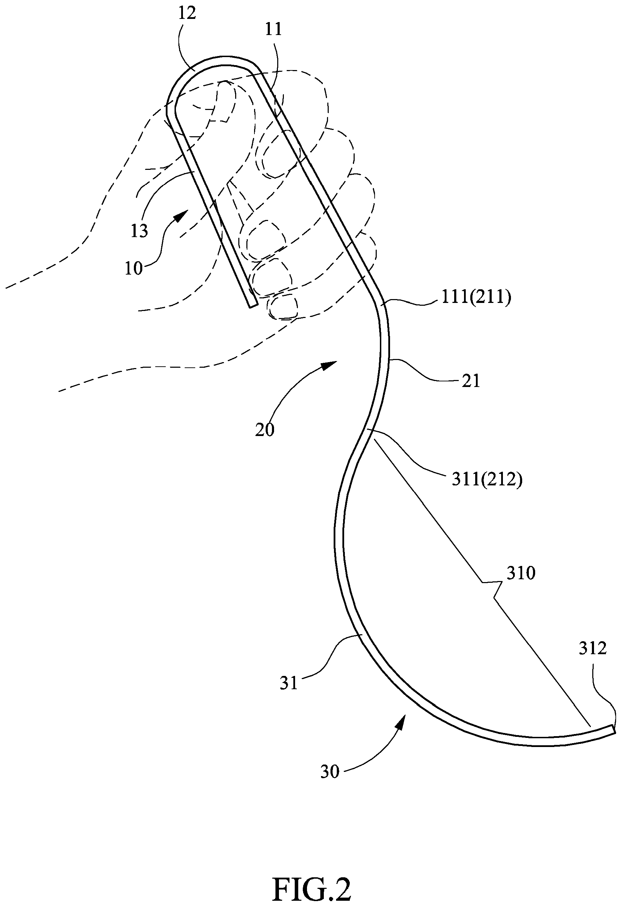

The handle 10 includes a main portion 11, an extension 13 substantially parallel to the main portion 11, and a bent interconnection 12 interconnecting the main portion 11 and the extension 13. In actual use, the length of the main portion 11 of the handle 10 is approximately the length of the palm of user's hand held. The connecting part 20 includes a curved connecting member 21 having an end 211 formed with a handle end 111 of the main portion 11, and the other end 212 formed with a curved portion 31 of the tongue lifting part 30. It is understood that the curved portion 31 of the tongue lifting part 30 is made from a plate-like strip having a uniform thickness. The curved portion 31 of the tongue lifting part 30 has a first end 311 and a second end 312, and the second end 312 is open. As viewed from the FIG. 2, the first end 311 (212) is a conversion point of two curves of the curved portion 31 and the curved connecting member 21. It is also to be understood that the point of the first end 311 (212) is substantially existed in all the figures. Further, the curved portion 31 has a width defined by two substantially parallel edges 316 (see FIG. 1).

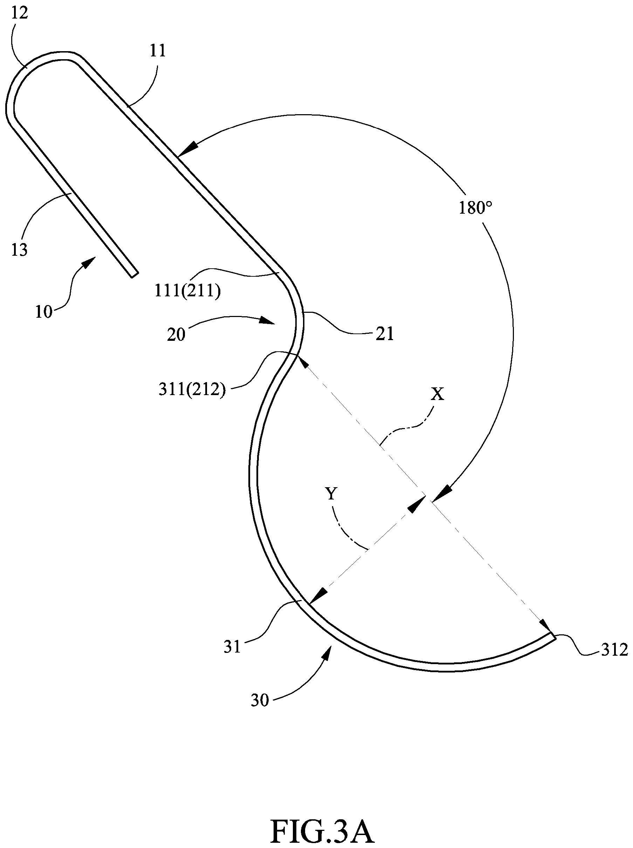

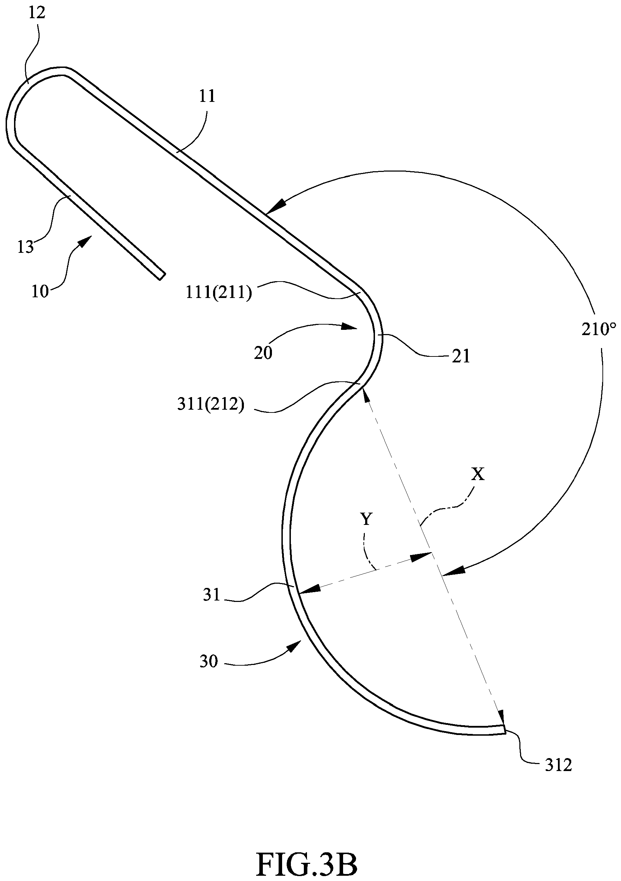

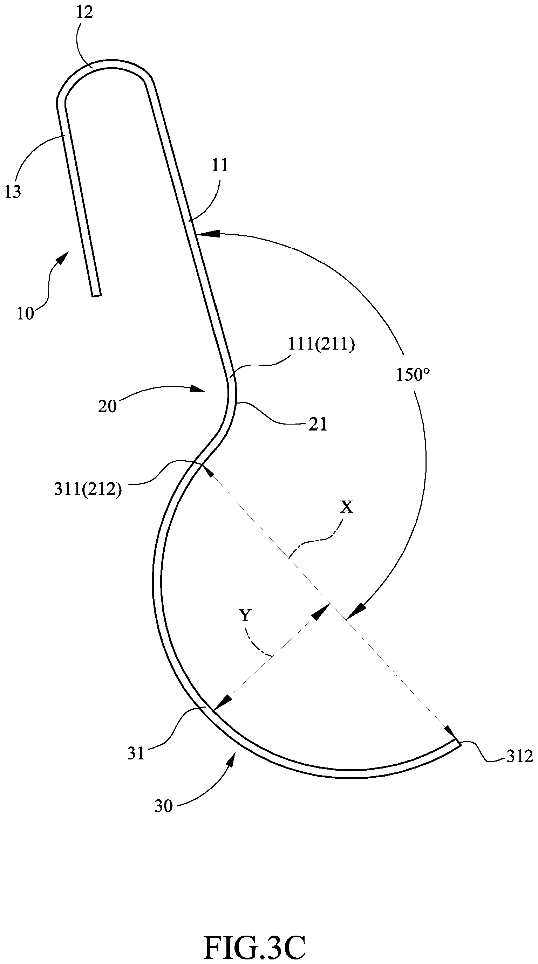

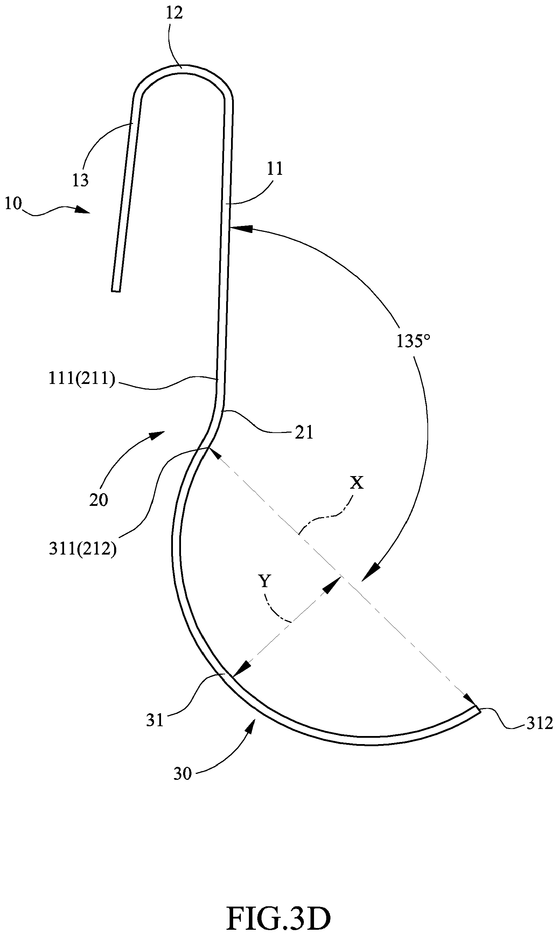

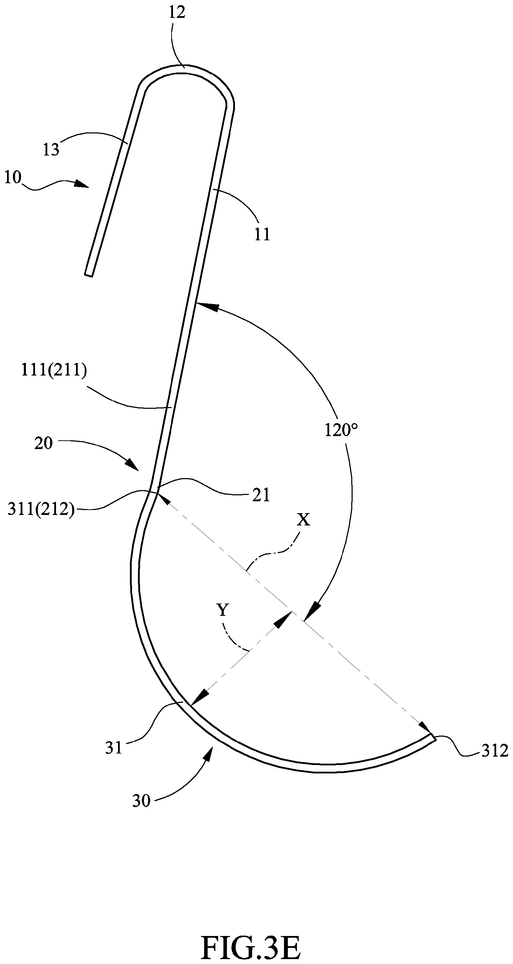

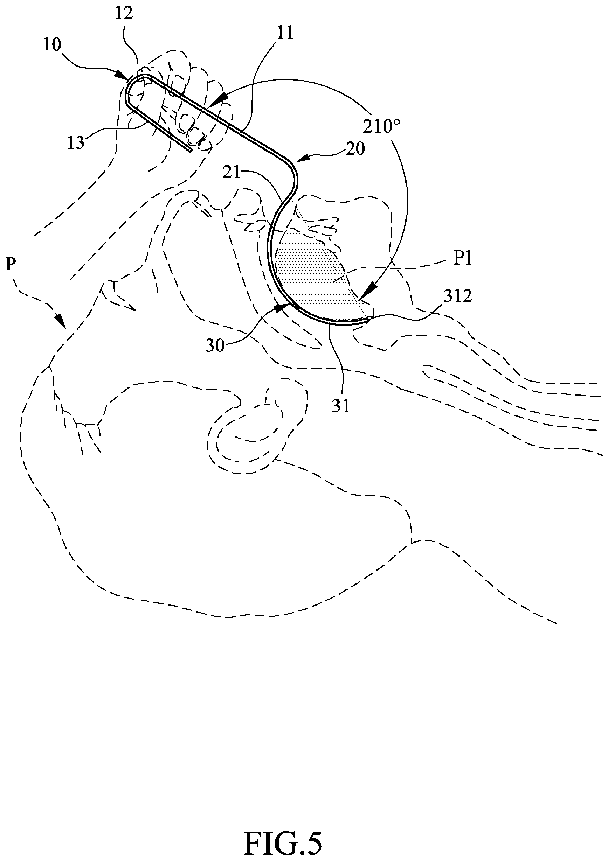

It is noted that the handle 10 may be shaped differently from the shown one in other embodiments. It is further noted that in addition to the connecting member 21 of the connecting part 20 can be shaped as a curved shape as shown in FIGS.3A to 3E. Alternatively, the connecting member 21 can be shaped other than a curve or a straight line in other embodiments depending on actual requirement. The tongue lifting part 30 can be made from a long strip of flat material by bending it into a predetermined shape, i.e. bending only along the long dimension 310 (see FIG. 2). Furthermore, it is noted that the curved portion 31 is made from a long dimension 310 and a short dimension 316, wherein the long dimension 310 is curved to form the curved portion 31 and the short dimension 316 is substantially straight, and wherein a subtense (X) is defined by joining the first end 311 and the second end 312 of the curved portion 31 and forms an angular relationship with the straight handle end 111 of the main portion 11, defining an angle ranged from 60 degrees to 210 degrees, respectively, as shown in FIGS. 3A-3E, and figure 5.

A maximum distance from the subtense (X) to the curved portion 31 is indicated by a dotted line is a depth (Y). It will be noted that the curved portion 31 of the tongue lifting part 30 is an arc of a circle or an arc of an ellipse itself, the length of the curved portion 31 is smaller than that of the semicircle or than that of the semi-ellipse itself. It means that the length of the subtense (X) is smaller than that of radius of circle or than that of major axis of the ellipse.

Preferably, the ratio of the length of the depth (Y) to that of the subtense (X) is in the range from 1/4 to 1/2, for example, the length of the subtense (X) used for an infant, a children and an adult are about 5-7 cm, 7-9 cm and 9-13 cm respectively.

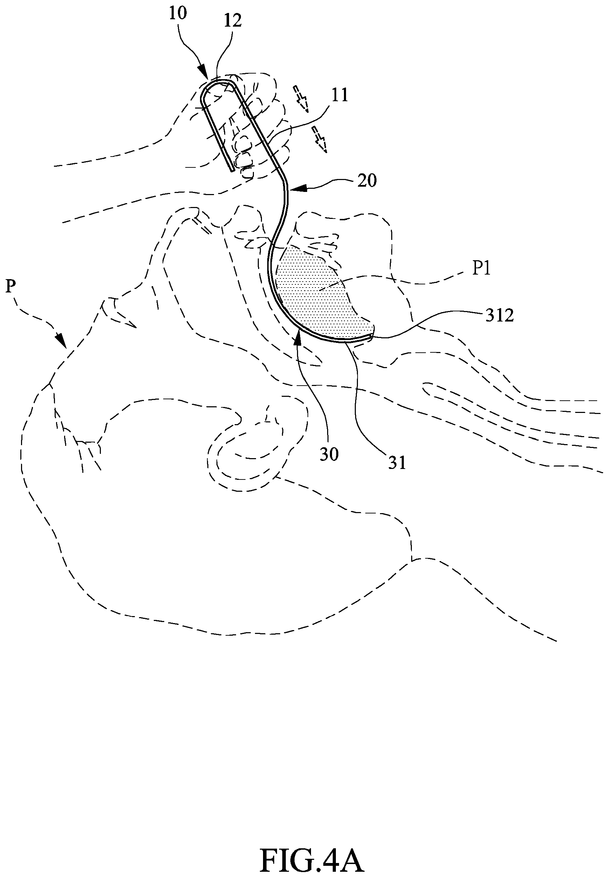

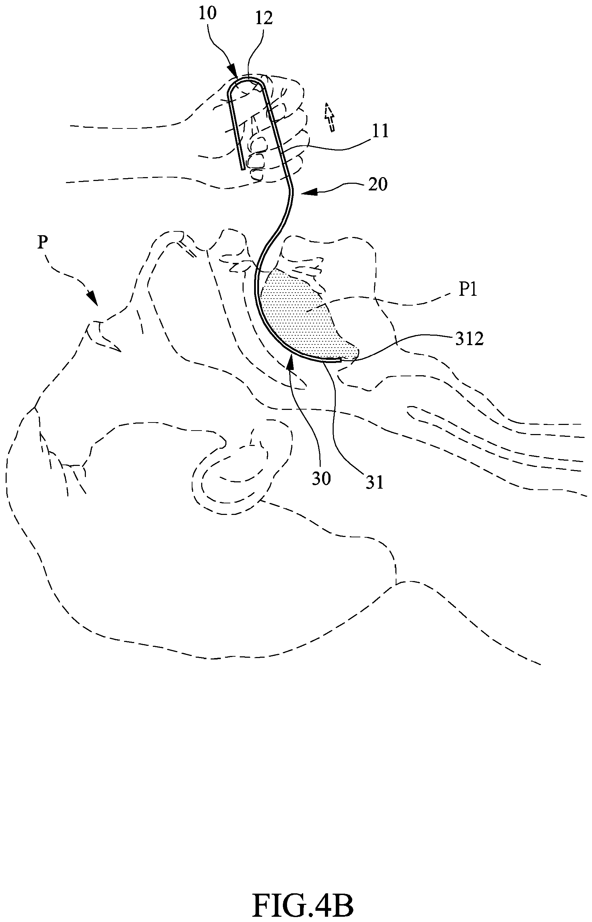

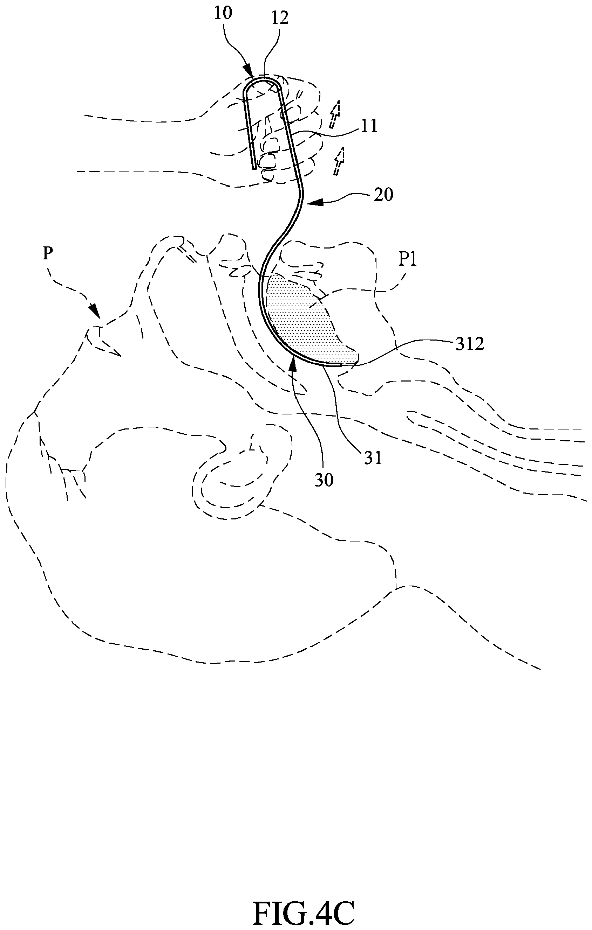

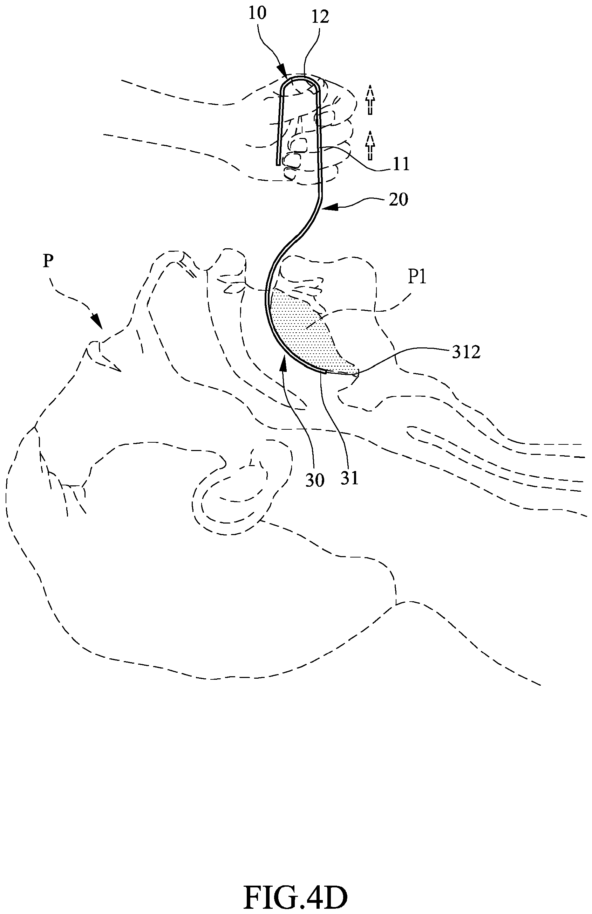

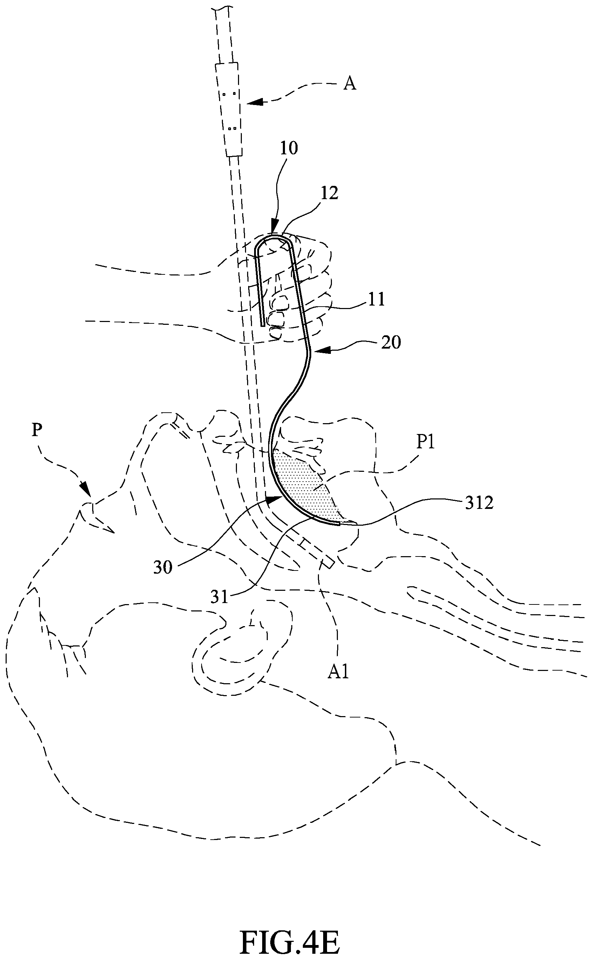

As shown in FIGS. 3A to 3E and 4A to 4E, the connecting part 20 is curved, the connecting member 21 is configured to made in a predetermined angle, and the tongue anteriorizer may be configured to shape in any other desired angle corresponding to the subtense (X), respectively. Operation of the first configuration of the first preferred embodiment of the tongue anteriorizer of the invention is described in detail below. A medical employee may use one hand to hold the main portion 11 of the handle 10 and insert the curved portion 31 of the tongue lifting part 30 into the mouth of a patient (P) until the second end 312 reaches the base of the posterior part of the tongue (P1) (see FIG. 4A). It is noted that the tongue (P1) can be moved anteriorly. Further, the posterior part of the tongue (P1) can be hooked by the second end 312 of the curved portion 31 prior to lifting the tongue (P1) (see FIG. 4B). Next, the employee may pull the main portion 11 of the handle 10 in a direction out of the mouth to cause the curved portion 31 of the tongue lifting part 30 to exert force on the tongue (P1) (see FIGS. 4C to 40). Thus, the tongue (P1) can be pulled by the curved portion 31 of the tongue lifting part 30 to move anteriorly along the top surface of the tongue (P1). As a result, the laryngopharyngeal space is open and increased, thereby allowing an endoscope (A) to easily insert into the mouth and positioning an end A1 of the endoscope (A) at a correct position in a natural manner (see FIG. 4E).

It is understood that whether insertion of the endoscope or endotracheal intubation is success or not depending on moving the tongue anteriorly and lifting it correctly. The size and shape of the curved portion 31 of the tongue lifting part 30 have been carefully calculated after considering ergonomics and the natural moving of the muscles of the tongue (P1). Therefore, the tongue anteriorizer of the invention is effective in the design and makes the device ergonomic.

Furthermore, as viewed from the FIGS. 4A-4E, it is noted that the curvature portion 31 of the tongue lifting part 30 is substantially two parallel edges 316 with a uniform thickness. When it is inserted into the mouth until it reaches a base of a posterior part of the tongue and hooked then pulled the tongue out very convenient in accordance with the moving muscle natural trend of the tongue without having any feeling hurt for the patient. The feature of the tongue lifting part 30 in the design might make the device ergonomic.

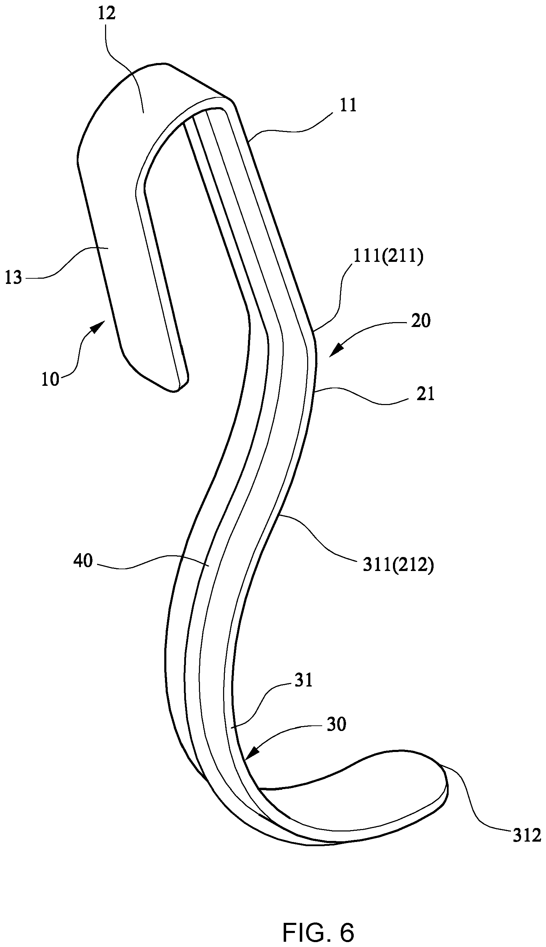

Referring to FIG. 6, a tongue anteriorizer is shown. The characteristics of the second preferred embodiment are substantially the same as that of the first preferred embodiment except the following: A rib 40 is formed on the middle portion of the back of the tongue anteriorizer to increase the rigidity of the tongue anteriorizr.

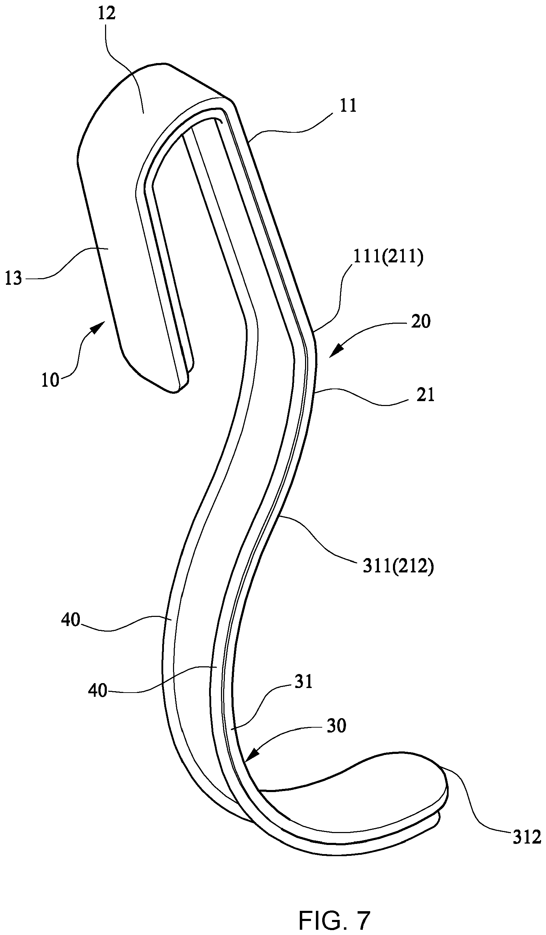

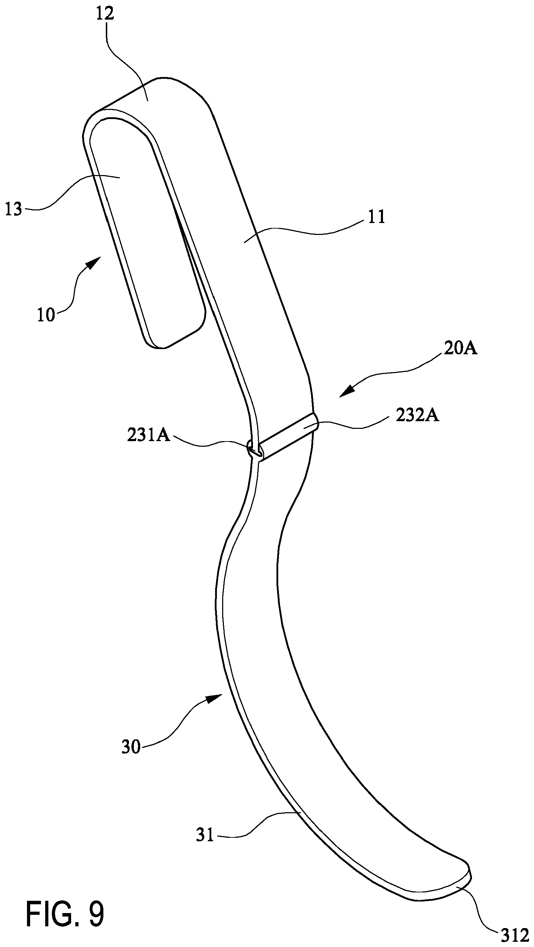

Referring to FIG. 7, 9, a tongue anteriorizer is shown. The characteristics in a second configuration of the second preferred embodiment are substantially the same as that of the first preferred embodiment except the following: Two ribs 40, 40 are formed on both sides of the back of the tongue anteriorizer to increase the rigidity of the tongue anteriorizr.

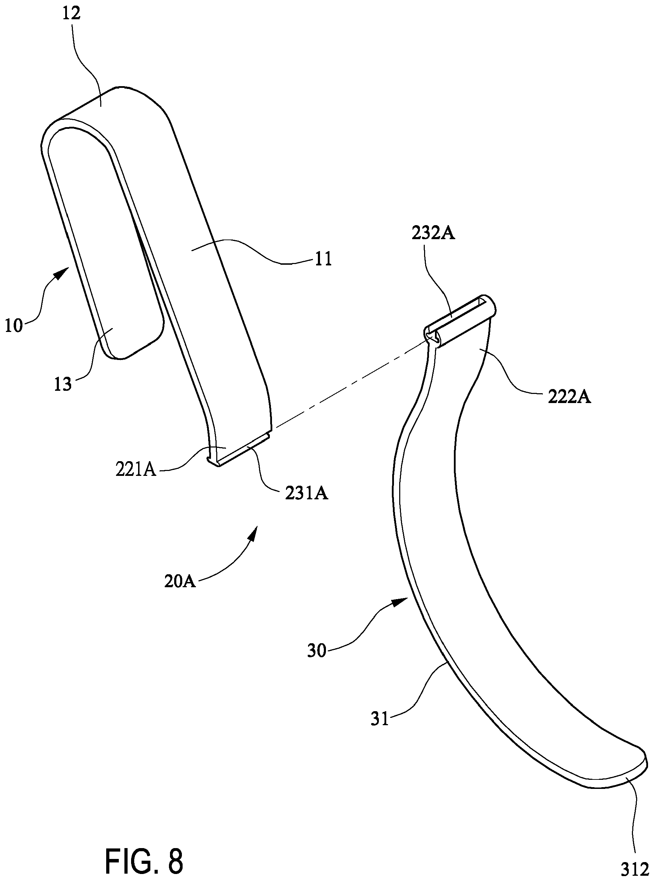

Referring to FIGS. 8 and 9, a tongue anteriorizer in accordance with the invention is shown. The characteristics of this embodiment are substantially the same as that of the previous embodiments except the following: A joint is formed on the connecting member of the connecting part 20A. The joint includes a rail 221A formed with the main portion 11 of the handle 10 and having two opposite elongated projections 231A, and a groove member 222A formed with the curved portion 31 of the tongue lifting part 30 and having a groove 232A. The groove 232A is shaped complimentarily to the rail 231A so that the rail 231A can be slidably inserted into the groove 232A to fasten the handle 10 and the tongue lifting part 30 together. This is a two-piece embodiment of the tongue anteriorizer of the invention and it facilitates manufacturing, assembly, and transport.

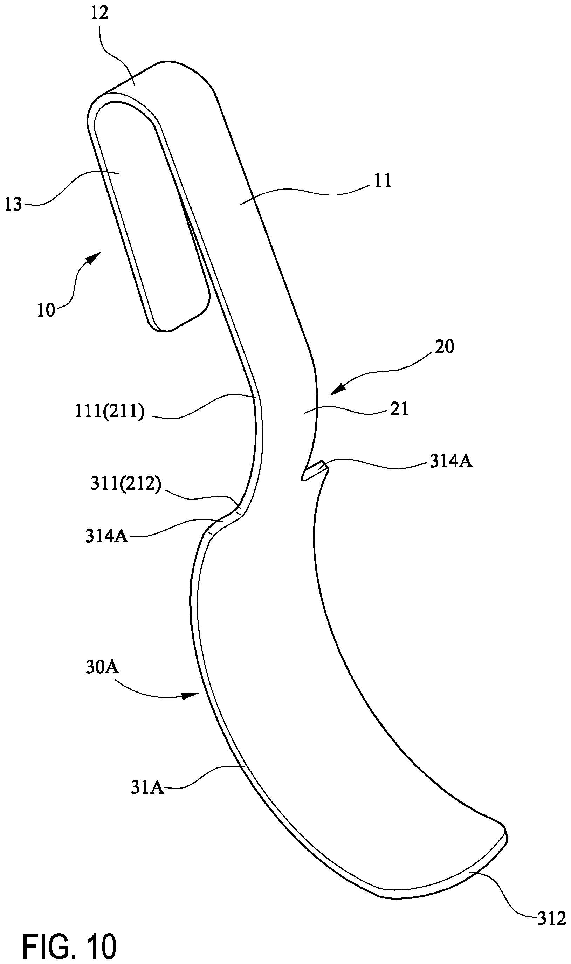

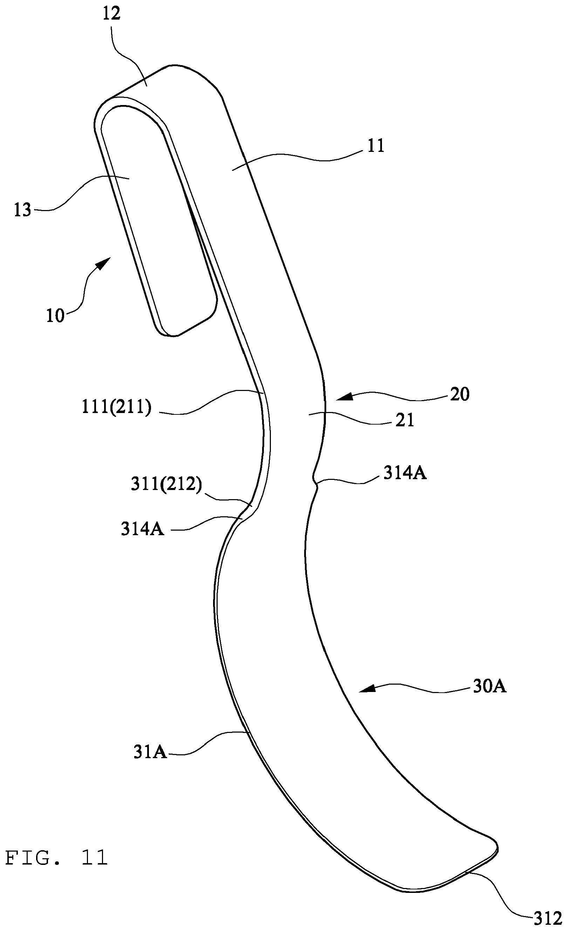

Referring to FIGS. 10 and 11, a tongue anteriorizer is shown. The characteristics of the fourth preferred embodiment are substantially the same as that of the first preferred embodiment except the following: In a first configuration, two opposite flat shoulders 314A are formed between the connecting member 21 of the connecting part 20 and the curved portion 31A of the tongue lifting part 30A so that width of the curved portion 31 A of the tongue lifting part 30A is greater than that of the connecting member 21 of the connecting part 20. In a second configuration, the shoulders 314A formed between the connecting member 21 of the connecting part 20 and the curved portion 31 A of the tongue lifting part 30A are two opposite inclined shoulders.

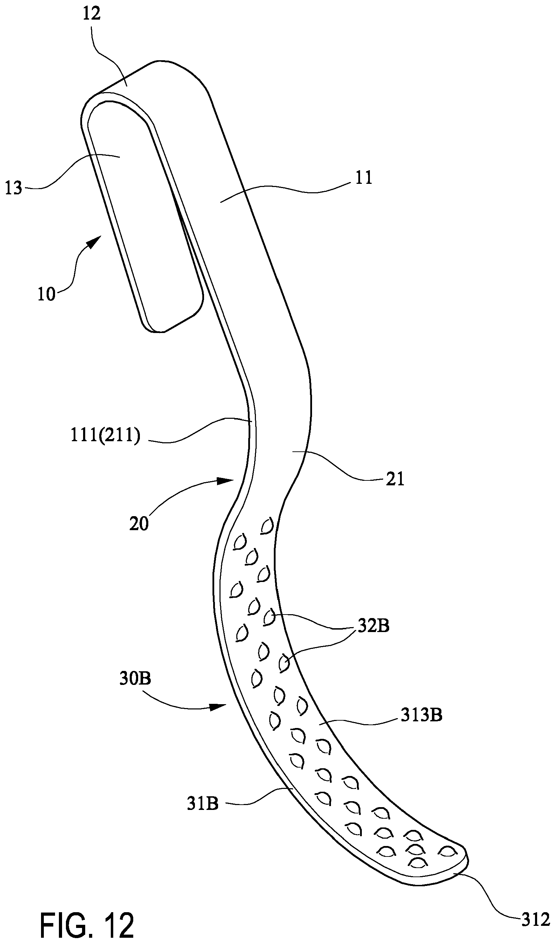

Referring to FIG. 12, a tongue anteriorizer is shown. The characteristics of the fifth preferred embodiment are substantially the same as that of the first preferred embodiment except the following: A plurality of protrusions 32B are formed on a long strip surface 313B of the curved portion 31B of the tongue lifting part 30B. The provision of the protrusions 32B can prevent the tongue lifting part 30B from slipping relative to the tongue.

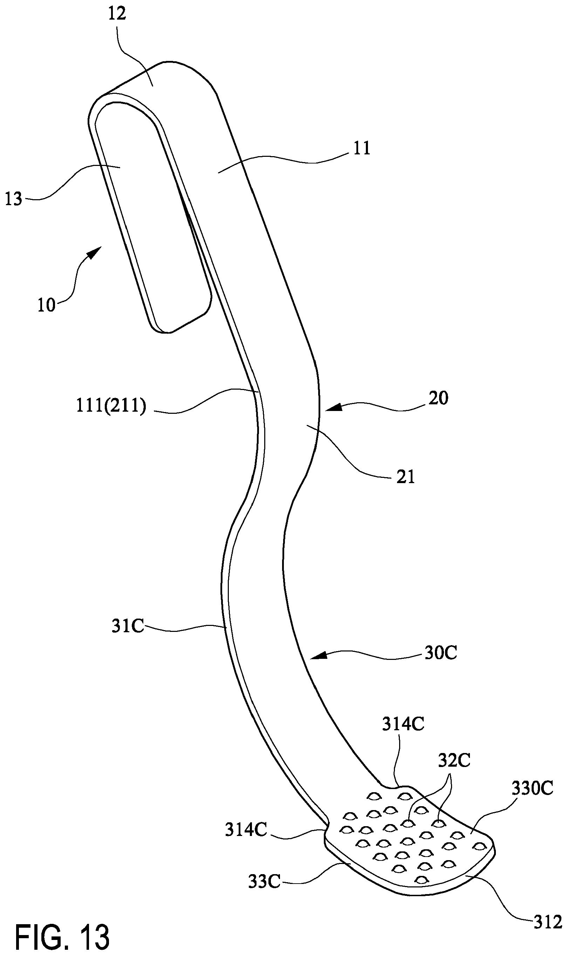

Referring to FIG. 13, a tongue anteriorizer is shown. The characteristics of the sixth preferred embodiment are substantially the same as that of the first preferred embodiment except the following: A plurality of protrusions 32C are formed on a long strip surface 330C of an enlargement 33C of the curved portion 31C of the tongue lifting part 30C adjacent to the second end 312. Two opposite inclined shoulders 314C are formed between the enlargement 33C and the curved portion 31 C of the tongue lifting part 30C.

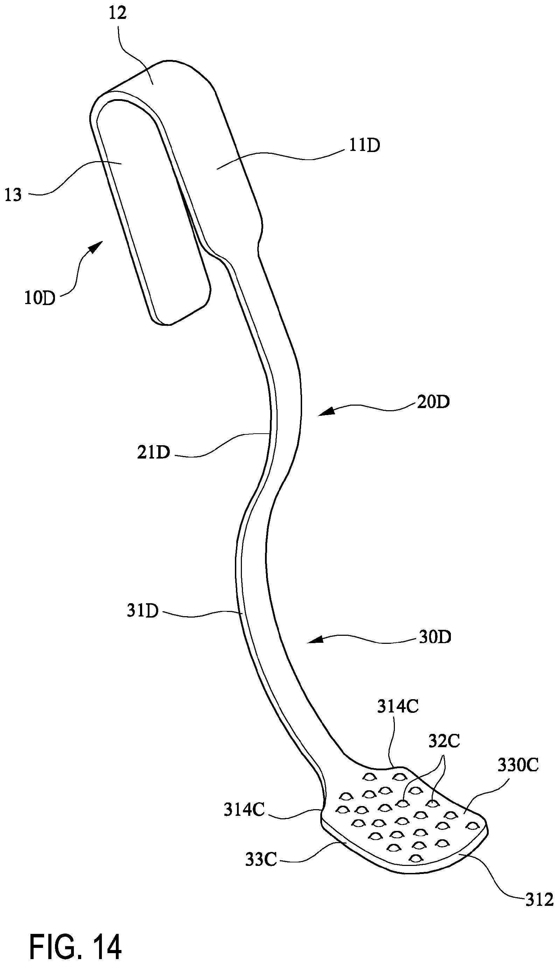

Referring to FIG. 14, a tongue anteriorizer is shown. The characteristics of the seventh preferred embodiment are substantially the same as that of the sixth preferred embodiment except the following. The handle 10D, the connecting part 20D and the tongue lifting part 30D are formed together by a plurality of narrower width portions wherein a wider portion 11D and a narrower portion are formed for the handle 10D, a narrower portion 21D is form for the connecting part 20D and the upper narrower portion 31D and the lower wider portion 33C are formed for the curved portion 312 of the tongue lifting part 30D so that the width of the curved portion 31D of the tongue lifting part 30D and the narrower portion 21D of the connecting part 20D are changed their width narrower than that of the above embodiments. It is to be understood that the optimum values of the slightly flexible deformation of the above-mentioned narrower portions for operating the tongue anteriorizer are provided for the particular patient.

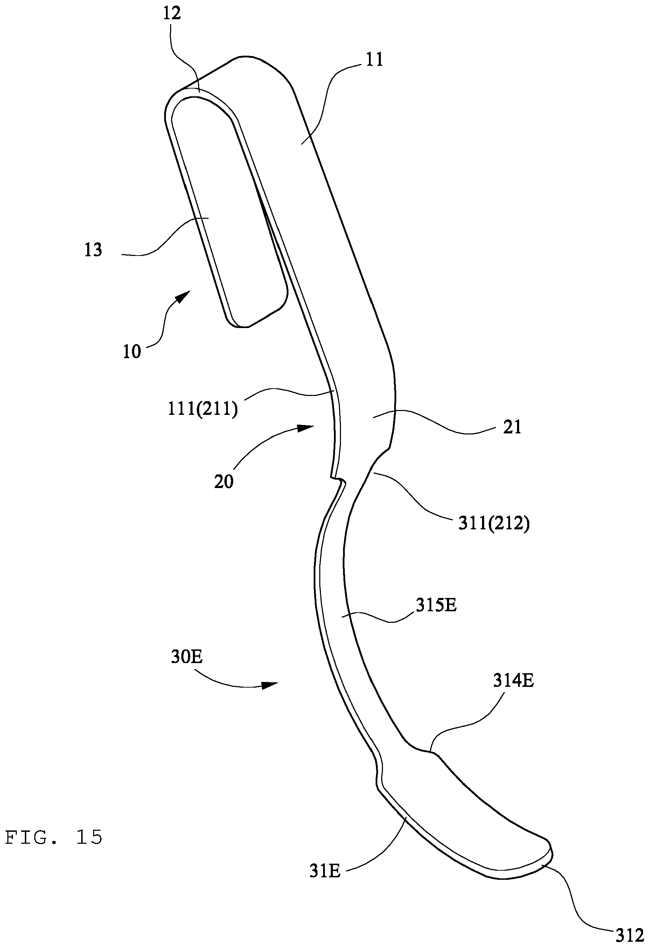

Referring to FIG. 15, a tongue anteriorizer is shown. The characteristics of the eighth preferred embodiment are substantially the same as that of the first preferred embodiment except the following. The curved portion 31E of the tongue lifting part 30E are formed by a long strip narrower portion 315E and a long strip wider portion 31E adjacent to the narrower portion 315E. Two opposite inclined shoulders 314E are formed between the narrower portion 315E and the wider portion 31E of the curved portion 312 of the tongue lifting part 30E. It is noted that the optimum value of the slightly flexible deformation for both of the connecting part 20 and the tongue lifting part 30E has been carefully calculated after considering ergonomics.

It is envisaged that when the tongue, especially its base, can be moved anteriorly and lifted, there will be no difficult intubation. The tongue anteriorizer can move the tongue anteriorly, open the laryngopharyngeal area, and make all endotracheal intubation simple, safe, easy and efficient.

## Claims

1. A tongue anteriorizer comprising:
a handle (10) including a main portion (11) having a handle end (111);
a tongue lifting part (30) including an arc member (31) having a first part end (311) integrally joined to the second end (212) of a connecting part (20) and a second part end (312), the tongue lifting part (30) being made from a plate-like strip having a uniform thickness and configured such that, in use, the second part end (312) can reach a base of the posterior part of a patient's tongue (P1) for hooking the posterior part, lifting the tongue and pulling the tongue anteriorly; and
the connecting part (20) including a connecting member (21) having a first end (211) formed with the handle end (111) of the main portion (11) of the handle (10) and a second end (212) formed with the first part end (311) of the arc member (31), wherein the connecting member (21) is a curve oriented in a direction opposite to a curved direction of the tongue lifting part (30) and the curved length of the connecting member (21, 21D) is less than one-third of the curved length of the arc member (31, 31A, 31B, 31 C, 31D, 31E),
wherein a subtense (X) is defined by a line connecting the first part end (311) and the second part end (312) of the arc member (31) and the main portion (11) is configured at a predetermined angle with respect to the subtense (X),
wherein the predetermined angle is individually formed between 60-degrees to 210-degrees for each tongue anteriorizer, and
wherein the maximum length of a line from the arc member (31) to the subtense (X) and perpendicular thereto is a length of a line(Y) and ratio of the length (Y) to the length of the subtense (X) is 1/4 to 1/2, the tongue anteriorizer being **characterized in that**
the handle (10) and tongue lifting part (30) are separable and the
connecting part (20A) comprises a rail (221A) that includes two opposite elongated projections (231A) and a groove member (232A) that includes a groove shaped complementarily to the rail (221A),
wherein the projections (231A) are formed on the first end of the connecting part (20A) that is formed with the main portion (11) of the handle (10) and the groove (232A) is formed on the second end of the connecting part (20A) that is formed with the arc member (31) of the tongue lifting part (30),
wherein the projections (231A) are formed as a projection element, and
wherein the groove (232A) is formed as an elongated concave element shaped complementarily to the projection element so that the projection element is configured to be insertable into the concave element to form connect the handle and tongue lifting part.

2. The tongue anteriorizer of claim 1, wherein the width of the arc member (31A) is greater than the width of the connecting member (21).

3. The tongue anteriorizer of claim 1, further comprising a first side, a second side and at least one rib (40), wherein the arc member (31) has a concave surface on the first side and the at least one rib (40) is arranged on the second side of the tongue anteriorizer for structural strength enhancement.

4. The tongue anteriorizer of claim 1, wherein an enlargement of a curved portion (31A) formed by an inclined shoulder (314A, 314C, 314E) adjacent to the second part end (312) is wider than a width of the curved portion (31A, 31C, 31D, 31E) of the tongue lifting part (30A, 30C, 30D, 30E) and, preferably, a plurality of protrusions (32) are formed on a longitudinal strip surface (330C) of an enlargement of the curved portion (31C, 31D) of the tongue lifting part (30C, 30D).

5. The tongue anteriorizer of claim 1, wherein the arc member (30B, 30C, 30D) includes a plurality of protrusions (32B, 32C).

6. The tongue anteriorizer according to claim 1, wherein the tongue lifting part (30) is an arc of a circle and the length of the subtense (X) is smaller than that of the diameter of the circle.

7. The tongue anteriorizer according to claim 1, wherein the tongue lifting part (30) is an arc of an ellipse and the length of the subtense (X) is smaller than that of the major axis of the ellipse.

8. The tongue anteriorizer according to claim 1, wherein the length of the subtense (X) used for an infant, a child and an adult is about 5-7 cm, 7-9 cm and 9-13 cm respectively.

9. The tongue anteriorizer of claim 1, wherein the width of the arc member (31) is greater than the width of the main portion (11).

## Patentansprüche

1. Zungenanteriorisator, umfassend:
einen Griff (10) einschließlich eines Hauptabschnitts (11), der ein Griffende (111) aufweist;
ein Zungenanhebeteil (30) einschließlich eines Bogenelements (31), das ein erstes Teilende (311), das einstückig mit dem zweiten Ende (212) eines Verbindungsteils (20) verbunden ist, und ein zweites Teilende (312) aufweist, wobei das Zungenanhebeteil (30) aus einem plattenartigen Streifen mit einer gleichmäßigen Dicke hergestellt und so konfiguriert ist, dass das zweite Teilende (312) im Gebrauch eine Basis des hinteren Teils der Zunge (P1) eines Patienten erreichen kann, um den hinteren Teil einzuhaken, die Zunge anzuheben und die Zunge nach vorne zu ziehen; und
das Verbindungsteil (20) ein Verbindungselement (21) mit einem ersten Ende (211), das mit dem Griffende (111) des Hauptabschnitts (11) des Griffs (10) ausgebildet ist, und einem zweiten Ende (212), das mit dem ersten Teilende (311) des Bogenelements (31) ausgebildet ist, enthält,
wobei das Verbindungselement (21) eine Kurve ist, die in einer Richtung entgegengesetzt zu einer gekrümmten Richtung des Zungenhebeteils (30) ausgerichtet ist, und die gekrümmte Länge des Verbindungselements (21, 21D) weniger als ein Drittel der gekrümmten Länge des Bogenelements (31, 31A, 31B, 31C, 31D, 31E) beträgt,
wobei eine Unterlänge (X) durch eine Linie definiert ist, die das erste Teilende (311) und das zweite Teilende (312) des Bogenelements (31) verbindet, und der Hauptabschnitt (11) unter einem vorbestimmten Winkel in Bezug auf die Unterlänge (X) konfiguriert ist,
wobei der vorbestimmte Winkel individuell zwischen 60 Grad und 210 Grad für jeden Zungenanteriorisator gebildet wird, und
wobei die maximale Länge einer Linie von dem Bogenelement (31) zu der Unterlänge (X) und senkrecht dazu eine Länge einer Linie (Y) ist und das Verhältnis der Länge (Y) zu der Länge der Unterlänge (X) 1/4 bis 1/2 beträgt, wobei der Zungenanteriorisator **dadurch gekennzeichnet ist, dass**
der Griff (10) und das Zungenhebeteil (30) trennbar sind und das Verbindungsteil (20A) eine Schiene (221A) umfasst, die zwei gegenüberliegende längliche Vorsprünge (231A) und ein Nutelement (232A) enthält, das eine komplementär zur Schiene (221A) geformte Nut enthält,
wobei die Vorsprünge (231A) an dem ersten Ende des Verbindungsteils (20A) ausgebildet sind, das mit dem Hauptabschnitt (11) des Griffs (10) ausgebildet ist, und die Nut (232A) an dem zweiten Ende des Verbindungsteils (20A) ausgebildet ist, das mit dem Bogenelement (31) des Zungenhebeteils (30) ausgebildet ist,
wobei die Vorsprünge (231A) als ein Vorsprungelement ausgebildet sind, und wobei die Nut (232A) als ein längliches konkaves Element ausgebildet ist, das komplementär zu dem Vorsprungelement geformt ist, so dass das Vorsprungelement so konfiguriert ist, dass es in das konkave Element einführbar ist, um den Griff und das Zungenhebeteil zu formzuverbinden.

2. Zungenanteriorisator gemäß Anspruch 1, wobei die Breite des Bogenelements (31A) größer ist als die Breite des Verbindungselements (21).

3. Zungenanteriorisator gemäß Anspruch 1, ferner umfassend eine erste Seite, eine zweite Seite und mindestens eine Rippe (40), wobei das Bogenelement (31) auf der ersten Seite eine konkave Oberfläche aufweist und die mindestens eine Rippe (40) auf der zweiten Seite des Zungenanteriorisators zur Verbesserung der strukturellen Festigkeit angeordnet ist.

4. Zungenanteriorisator gemäß Anspruch 1, wobei eine Erweiterung eines gekrümmten Abschnitts (31A), der durch eine schräge Schulter (314A, 314C, 314E) neben dem zweiten Teilende (312) gebildet wird, breiter ist als eine Breite des gekrümmten Abschnitts (31A, 31C, 31D, 31E) des Zungenhebeteils (30A, 30C, 30D, 30E) und vorzugsweise eine Vielzahl von Vorsprüngen (32) auf einer Längsstreifenoberfläche (330C) einer Erweiterung des gekrümmten Abschnitts (31C, 31D) des Zungenhebeteils (30C, 30D) ausgebildet sind.

5. Zungenanteriorisator gemäß Anspruch 1, wobei das Bogenelement (30B, 30C, 30D) eine Vielzahl von Vorsprüngen (32B, 32C) aufweist.

6. Zungenanteriorisator gemäß Anspruch 1, wobei das Zungenhebeteil (30) ein Kreisbogen ist und die Länge der Unterlänge (X) kleiner ist als der Durchmesser des Kreises.

7. Zungenanteriorisator gemäß Anspruch 1, wobei das Zungenhebeteil (30) ein Ellipsenbogen ist und die Länge der Unterlänge (X) kleiner ist als die der Hauptachse der Ellipse.

8. Zungenanteriorisator gemäß Anspruch 1, wobei die Länge der Unterlänge (X), die für einen Säugling, ein Kind und einen Erwachsenen verwendete wird, etwa 5-7 cm, 7-9 cm bzw. 9-13 cm beträgt.

9. Zungenanteriorisator gemäß Anspruch 1, wobei die Breite des Bogenelements (31) größer ist als die Breite des Hauptabschnitts (11).

## Revendications

1. Un dispositif d'antériorisation de langue comprenant :
un manche (10) comprenant une partie principale (11) ayant une extrémité de manche (111) ;
une partie (30) de soulèvement de langue comprenant un élément arqué (31) ayant une première extrémité de partie (311) solidairement reliée à la deuxième extrémité (212) de la partie de liaison (20) et une deuxième extrémité de partie (312), la partie (30) de soulèvement de langue étant constituée d'une bande en forme de plaque ayant une épaisseur uniforme et configurée de telle sorte que, en cours d'utilisation, la deuxième extrémité de partie (312) soit apte à atteindre une base de la partie postérieure de la langue (P1) d'un patient pour accrocher la partie postérieure, soulevant la langue et tirant la langue de façon à l'anterioriser ; et
la partie de liaison (20) comprenant un élément de liaison (21) ayant une première extrémité (211) formée avec l'extrémité de poignée (111) de la partie principale (11) de la poignée (10) et une deuxième extrémité (212) formée avec la première extrémité de partie (311) de l'élément arqué (31), l'élément de liaison (21) étant une courbe orientée dans une direction opposée à une direction incurvée de la partie (30) de soulèvement de langue et la longueur incurvée de l'élément de liaison (21, 21D) est inférieure à un tiers de la longueur incurvée de l'élément arqué (31, 31A, 31B, 31C, 31D, 31E),
un sous-tendant (X) est défini par une ligne reliant la première extrémité de partie (311) et la deuxième extrémité de partie (312) de l'élément arqué (31) et la partie principale (11) est configurée selon un angle prédéterminé par rapport au sous-tendant (X),
l'angle prédéterminé étant formé individuellement entre 60 degrés et 210 degrés pour chaque dispositif d'antériorisation de langue, et
la longueur maximale d'une ligne allant de l'élément arqué (31) au sous-tendant (X) et perpendiculaire à celui-ci étant une longueur de ligne (Y) et le rapport de la longueur (Y) à la longueur du sous-tendant (X) est de 1/4 à 1/2, le dispositif d'antériorisation de langue étant **caractérisé en ce que**
la poignée (10) et la partie (30) de soulèvement de langue sont séparables et la partie de liaison (20A) comprend un rail (221A) qui comprend deux saillies allongées opposées (231A) et un élément formant rainure (232A) qui comprend une rainure de forme complémentaire au rail (221A),
les saillies (231A) étant formées sur la première extrémité de la partie de liaison (20A) qui est formée avec la partie principale (11) de la poignée (10) et la rainure (232A) est formée sur la deuxième extrémité de la partie de liaison (20A) qui est formé avec l'élément arqué (31) de la partie (30) de soulèvement de langue,
les saillies (231A) étant sous la forme d'un élément de saillie, et
la rainure (232A) étant formée sous la forme d'un élément concave allongé façonné de manière complémentaire à l'élément de saillie de sorte que l'élément de saillie soit configuré de façon à être insérable dans l'élément concave afin de former une liaison entre la poignée et la partie de soulèvement de langue.

2. Le dispositif d'antériorisation de langue selon la revendication 1, dans lequel la largeur de l'élément arqué (31A) est supérieure à la largeur de l'élément de liaison (21).

3. Le dispositif d'antériorisation de langue selon la revendication 1, comprenant en outre un premier côté, un deuxième côté et au moins une nervure (40), l'élément arqué (31) ayant une surface concave sur le premier côté et ladite au moins une nervure (40) étant agencée sur le deuxième côté du dispositif d'antériorisation de langue pour améliorer la résistance structurelle.

4. Le dispositif d'antériorisation de langue selon la revendication 1, dans lequel un élargissement d'une partie incurvée (31A) formée par un épaulement incliné (314A, 314C, 314E) adjacent à la deuxième extrémité de partie (312) est plus large qu'une largeur de la partie incurvée (31A, 31C, 31D, 31E) de la partie (30A, 30C, 30D, 30E) de soulèvement de langue, et, de préférence, une pluralité de saillies (32) est formée sur une surface de bande longitudinale (330C) d'un élargissement de la partie incurvée (31C, 31D) de la partie de soulèvement de langue (30C, 30D).

5. Le dispositif d'antériorisation de langue selon la revendication 1, dans lequel l'élément arqué (30B, 30C, 30D) comprend une pluralité de saillies (32B, 32C).

6. Le dispositif d'antériorisation de langue selon la revendication 1, dans lequel la partie (30) de soulèvement de langue est un arc de cercle et la longueur du sous-tendant (X) est inférieure à celle du diamètre du cercle.

7. Le dispositif d'antériorisation de langue selon la revendication 1, dans lequel la partie (30) de soulèvement de langue est un arc d'ellipse et la longueur du sous-tendant (X) est inférieure à celle du grand axe de l'ellipse.

8. Le dispositif d'antériorisation de langue selon la revendication 1, dans lequel la longueur du sous-tendant (X) utilisé pour un nourrisson, un enfant et un adulte est d'environ 5 à 7 cm, 7 à 9 cm et 9 à 13 cm respectivement.

9. Le dispositif d'antériorisation de langue selon la revendication 1, dans lequel la largeur de l'élément arqué (31) est supérieure à la largeur de la partie principale (11).
